# EUROPEAN PATENT SPECIFICATION

(11) **EP 4 240 328 B1**
(45) Date of publication and mention of the grant of the patent: **22.04.2026**
(21) Application number: 21889696.7
(22) Date of filing: 08.11.2021
(51) Int. Cl.: A61K 9/127, A61K 38/00, A61K 47/69, A61P 31/14

(54) **LIPOSOME, ESPECIALLY FOR USE IN THE TREATMENT OF COVID-19**
LIPOSOM, INSBESONDERE ZUR VERWENDUNG BEI DER BEHANDLUNG VON COVID-19
LIPOSOME, DESTINÉ EN PARTICULIER À ÊTRE UTILISÉ DANS LE TRAITEMENT DE LA COVID-19

(30) Priority: 08.11.2020 PL 43592120
(43) Date of publication of application: 13.09.2023
(73) Proprietor: Acellmed Sp. z o.o., 41-800 Zabrze (PL)
(72) Inventor: SIKORSKI, Aleksander F., 55-114 Rogoz (PL); KONKA, Adam Piotr, 00-132 Warszawa (PL); KULICZKOWSKI, Kazimierz, 51-107 Wroclaw (PL)
(74) Representative: Witek, Rafal
(86) International application number: PCT/PL2021/050078
(87) International publication number: WO 2022/098250

(56) References cited:
- CN-A- 111 529 685
- US-A1- 2009 092 548
- NALLAMOTHU R. ET AL.: "A TUMOR VASCULATURE TARGETED LIPOSOME DELIVERY SYSTEM FOR COMBRETASTATIN A4: DESIGN, CHARACTERIZATION, AND IN VITRO EVAULATION", AAPS PHARMSCITECH, SPRINGER NEW YORK LLC, US, vol. 7, no. 2, 7 April 2006 (2006-04-07), pages E01 - E10, XP008067953, ISSN: 1530-9932, DOI: 10.1208/PT070232
- YANG J. ET AL.: "Molecular interaction and inhibition of SARS-CoV-2 binding to the ACE2 receptor", 11 September 2020 (2020-09-11), England, pages 1 - 10, XP055799014, Retrieved from the Internet <URL:https://www.nature.com/articles/s41467-020-18319-6.pdf> DOI: 10.1038/s41467-020-18319-6
- HAN D.P. ET AL.: "Identification of critical determinants on ACE2 for SARS-CoV entry and development of a potent entry inhibitor", VIROLOGY, ELSEVIER, AMSTERDAM, NL, vol. 350, no. 1, 20 June 2006 (2006-06-20), pages 15 - 25, XP024896563, ISSN: 0042-6822, [retrieved on 20060620], DOI: 10.1016/J.VIROL.2006.01.029
- YANG JINSUNG, PETITJEAN SIMON, KOEHLER MELANIE, ZHANG QINGRONG, DUMITRU ANDRA C, CHEN WENZHANG, DERCLAYE SYLVIE, VINCENT STéP: "Molecular interaction and inhibition of SARS-CoV-2 binding to the ACE2 receptor", NATURE COMMUNICATIONS, ENGLAND, 11 September 2020 (2020-09-11), England , pages 1 - 10, XP055799014, DOI: 10.1038/s41467-020-18319-6
- HAN, D.P. ; PENN-NICHOLSON, A. ; CHO, M.W.: "Identification of critical determinants on ACE2 for SARS-CoV entry and development of a potent entry inhibitor", VIROLOGY, ELSEVIER, AMSTERDAM, NL, vol. 350, no. 1, 20 June 2006 (2006-06-20), AMSTERDAM, NL , pages 15 - 25, XP024896563, ISSN: 0042-6822, DOI: 10.1016/j.virol.2006.01.029

### List of sequences

<110> Acellmed Sp. z o.o.
<120> Liposom, for treatment against COVID-19
<130> PK/7820/RW
<160> 3
<170> PatentIn version 3.5
<210> 1
<211> 24
<212> PRT
<213> artificial
<220>
<223> peptide-1
<400> 1
   Glu Glu Gln Ala Lys Thr Phe Leu Asp Lys Phe Asn His Glu Ala Glu
   1 5 10 15
   Asp Leu Phe Tyr Gln Ser Ser Cys
   20
<210> 2
<211> 8
<212> PRT
<213> artificial
<220>
<223> peptide-2
<400> 2
   Leu Gly Lys Gly Asp Phe Arg Cys
   1 5
<210> 3
<211> 32
<212> PRT
<213> artificial
<220>
<223> prptide-3
<400> 3
   Glu Glu Gln Ala Lys Thr Phe Leu Asp Lys Phe Asn His Glu Ala Glu
   1 5 10 15
   Asp Leu Phe Tyr Gln Ser Ser Gly Leu Gly Lys Gly Asp Phe Arg Cys
   20 25 30

## Description

The object of the invention is a pharmaceutical preparation containing liposomes which have, on their surface, a peptide with the sequence of the SARS-Cov-2 virus receptor (severe acute respiratory syndrome coronavirus-2), so-called ACE2 (angiotensin converting enzyme-2) found on the surface of human cells (respiratory epithelium) and its use in the treatment of acute conditions caused by SARS-Cov-2 virus infection (COVID-19).

Plasma from recovered patients appears to be the only effective drug so far for the first phase (up to day 14 after symptom onset) of acute COVID-19 cases. The mechanism of action remains unknown, but is suggested to be based on immediate virus neutralization and antibody-dependent cytotoxicity as well as phagocytosis. Moreover, such plasma may result in decreased macrophage activation and systemic inhibition of the hyper-inflammatory state, the so-called "cytokine storm". However, the problem remains the very low availability of such plasma and the necessary large doses - about 200 ml of such plasma is used per patient, which is basically obtained from the entire portion of blood obtained from a single donor.

On the other hand, the mechanism of binding of the SARS-CoV-2 spike protein to the ACE2 (angiotensin converting enzyme-2) receptor protein of human cells is well understood. These are two sections of the ACE2 structure distant in sequence (amino acid residues 22-44 351-357) but located close in the spatial structure of this protein. The studies published by other teams show that the peptide obtained by combining the two sequences with a glycine residue inhibits the interaction of the SARS-CoV virus surface protein S with the ACE2 receptor.

The aim of the invention is to develop a drug for the treatment of COVID-19 and as an alternative to the plasma of the recovered patients.
Unexpectedly, such a defined problem is solved in the present invention.

An object of the invention is a liposome comprising a shell containing 65-70 mol% hydrogenated soy phosphatidylcholine (HSPC), 10 mol% distearoylphosphatidylcholine (DSPC), 5.0 mol% dipalmitoylphosphatidylglycerol, 4.5 mol% DSPE-PEG, 10-15 mol% cholesterol and 0.5 mol% maleimide derivative of DSPE-PEG (DSPE-PEG-mal), wherein a peptide having an amino acid sequence of Sequence No 3 is attached to the PEG residue of the DSPE-PEG molecule.

Another object of the invention is a liposome according to the invention as defined above for use in the treatment of COVID-19.

Another object of the invention is a pharmaceutical preparation comprising a liposome according to the invention as defined above.

An advantageous implementation of the invention is a liposome preparation comprising on its surface a peptide conjugated to the PEG residue of DSPE-PEG with the sequence of the coronavirus receptor, ACE2, advantageously residues: 22-44-G-351-357-C, which has a negatively charged lipid in the composition and contains 65-70 mol% hydrogenated soybean phosphatidylcholine (HSPC), 10 mol% distearoylphosphatidylcholine (DSPC), 5.0 mol% dipalmitoylphosphatidylglycerol, 4.5 mol% DSPE-PEG, 0.5 mol% maleimide derivative of DSPE-PEG (DSPE-PEG-mal), and 10-15 mol% cholesterol. The molecule responsible for the interaction with the coronavirus spike protein are peptides with the ACE2 receptor sequence of the protein, namely: peptide 3 (residues 22-44-G-351-357-C with sequence EEQAKTFLDKFNHEAEDLFYQSS-G-LGKGDFRC). Complementing the above peptides with a carboxyterminal cysteine residue will facilitate binding of the peptide to a maleimide derivative of DSPE-PEG.

A further embodiment of the invention is the use of the liposomal preparation defined above for the treatment of acute conditions of infection with COVID-19.

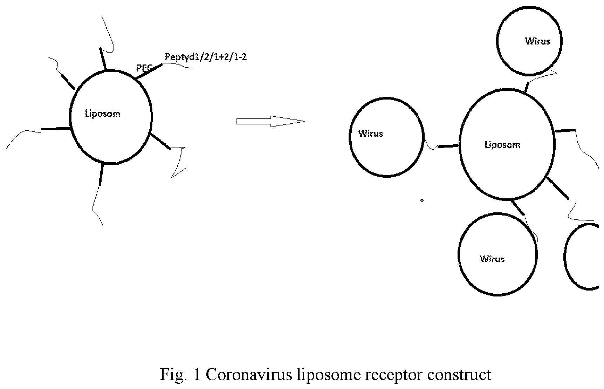

The proposed nano-therapeutic based on liposomes containing polyethylene glycol derivatives on the surface, to which the designed peptide is attached, i.e. a peptide having a specific linker connected sequence of both peptide-receptor elements . (22-44-G-351-257) (Fig. 1).

We expect that such an inhibitor nano-form not only competitively inhibits the binding of the virus to the cell surface, but also immobilizes it on the liposome surface (diameter of about 100 nm) and thus impedes the progression of infection. It is very likely that such a liposome exposing viruses on its surface activates the production of inactivating antibodies, as patients treated with plasma from recovered patients are characterized by a significant increase in the level of these antibodies.

Moreover, the presented liposome preparation is characterized by high stability during the months of storage and in contact with human serum, making it a privileged candidate for the development of a suitable pharmaceutical formulation.

The invention relates to liposomes containing on their surface polyethylene glycol derivatives to which an engineered peptide having the sequence of residues 22-44-G-351-357-C is attached. The nano-form of the inhibitor proposed according to the invention can not only competitively inhibit the binding of the virus to the cell surface, but should also immobilize it on the surface of the liposome (diameter of about 100 nm) and thus impede the progress of infection. In addition, the liposome exposing viruses on the surface, it should also induce the production of antibodies (in published studies it is shown that patients treated with plasma of recovered patients are also characterized by a significant increase in the level of their own antibodies inactivating the virus).

A drug formulation prepared in this manner solves several important problems associated with the treatment of acute COVID-19 conditions:
1. The drug can be produced on an industrial scale,
2. The drug is stable in long-term storage (min. 8 months),
3. The drug is not a blood product, so there is no risk of transmission of other diseases
4. The drug can be additionally enriched with other components encapsulated inside the liposome.

### Example 1. Preparation of a liposome according to the invention

To obtain 1 ml of liposome suspension containing 20 mg of lipids, chloroform solutions containing the following amounts of lipids are mixed:
13.0 mg (70 mol%) of hydrogenated soybean phosphatidylcholine (HSPC),
1.88 mg (10 mol%) distearoylphosphatidylcholine (DSPC),
0.88 mg (5 mol%) dipalmitoylphosphatidylglycerol (DPPG),
2.98 mg (4.5 mol%) DSPE-PEG,
0.34 mg (0.5 mol%) maleimide derivative of DSPE-PEG (DSPE-PEG-mal), and
0.92 mg (10 mol%) cholesterol

And then the solvent is removed by evaporation in a vacuum evaporator, after which the resulting lipid film is dried under reduced pressure (preferably using an oil pump) for 2 hours. Liposomes are formed by hydration of the film for 60 min with 1.0 ml of 50 mM HEPES, 100 mM NaCl, pH 7.2 at 64oC by light shaking with glass beads. After this time, the homogeneous suspension is extruded through Nucleopore (Whatman) membranes with diameters of 400, 200 and finally 100 nm successively in a pressure extruder maintaining a temperature of 64oC. The forcing through each membrane is repeated ten times. The obtained liposome suspension is homogeneous in particle diameter (120-130 nm) and zeta potential (the so-called polydispersity index, PDI is less than 0.1). These parameters are obtained by dynamic light scattering measurements and LDV (Laser Doppler Velocimetry) technique in Zeta-Sizer-Nano apparatus (Malvern Instruments).

The liposomes obtained in this way are incubated with peptide 3 with the sequence: EEQ AKT FLD KFN HEA EDL FYQ SSG LGK GDF RC, which contains one cysteine residue enabling the reaction with maleimide derivative of DSPE-PEG. Alternatively, the reaction is carried out with peptide 1 or 2. To ensure full saturation of the reactive groups, the number of moles of the peptide is twice as large as the number of moles of DSPE-PEG-mal. So in this case mol wt~3000 and mol wt DSPE-PEG-mal also about 3000, so 0.68 mg is used, adding 68 µl of a 10 mg/ml solution of peptide in 50 mM HEPES buffer, 100 mM NaCl, pH 7.2 to the liposome suspension and incubated at 4°C for 24 hours. Unbound peptide is removed by filtration on a 1.2 x25 cm column filled with Sepharose 4B gel and equilibrated with the above buffer. Chromatography can be performed at room temperature by collecting 0.5 ml fractions and reading the absorbance in a spectrophotometer at wavelengths of 330 (turbidity, liposomes) and 280 nm (peptide). The liposomes obtained in the void volume (first peak) are characterized in a ZetaSizer apparatus.

The preparation thus prepared is ready for biological activity analyses, primarily in a syncytium formation inhibition assay, which involves mixing cells, e.g. HEK293 transfected with a vector carrying the ACE2 receptor with HEK293 cells transfected with a vector carrying a sequence recognizing the SARS - COV2 virus protein S receptor.

## Claims

1. A liposome comprising a shell containing 65-70 mol% hydrogenated soy phosphatidylcholine (HSPC), 10 mol% distearoylphosphatidylcholine (DSPC), 5.0 mol% dipalmitoylphosphatidylglycerol, 4.5 mol% DSPE-PEG, 10-15 mol% cholesterol and 0.5 mol% maleimide derivative of DSPE-PEG (DSPE-PEG-mal) wherein a peptide having an amino acid Sequence 3 is attached to the PEG residue of the DSPE-PEG molecule.

2. A liposome as defined in claim 1 for use in the treatment of COVID-19.

3. A pharmaceutical preparation comprising a liposome as defined in claim 1.

## Patentansprüche

1. Liposom, umfassend eine Hülle, die 65-70 Mol-% hydriertes Soja-Phosphatidylcholin (HSPC), 10 Mol-% Distearoylphosphatidylcholin (DSPC), 5,0 Mol-% Dipalmitoylphosphatidylglycerol, 4,5 Mol-% DSPE-PEG, 10-15 Mol-% Cholesterin und 0,5 Mol-% Maleimid-Derivat von DSPE-PEG (DSPE-PEG-mal) enthält, wobei ein Peptid, das eine Aminosäuresequenz 3 aufweist, an den PEG-Rest des DSPE-PEG-Moleküls gebunden ist.

2. Liposom nach Anspruch 1 zur Verwendung bei der Behandlung von COVID-19.

3. Pharmazeutische Zubereitung, umfassend ein Liposom nach Anspruch 1.

## Revendications

1. Liposome comprenant une enveloppe contenant 65-70 %mol de phosphatidylcholine de soja hydrogénée (HSPC), 10 %mol de distéaroylphosphatidylcholine (DSPC), 5,0 %mol de dipalmitoylphosphatidylglycérol, 4,5 %mol de DSPE-PEG, 10-15 %mol de cholestérol et 0,5 %mol de dérivé maléimide de DSPE-PEG (DSPE-PEG-mal) dans lequel un peptide présentant une Séquence d'acides aminés 3 est fixé au résidu PEG de la molécule de DSPE-PEG.

2. Liposome tel que défini dans la revendication 1 destiné à être utilisé dans le traitement de la COVID-19.

3. Préparation pharmaceutique comprenant un liposome tel que défini dans la revendication 1.
